# EUROPEAN PATENT SPECIFICATION

(11) **EP 4 339 274 B1**
(45) Date of publication and mention of the grant of the patent: **26.03.2025**
(21) Application number: 22195394.6
(22) Date of filing: 13.09.2022
(51) Int. Cl.: C12M 1/107, C12M 1/34, C12M 1/36, C12M 1/00, C12M 1/26

(54) **METHOD FOR OPERATING A BIOPROCESS INSTALLATION FOR PRODUCTION OF A BIOPRODUCT**
VERFAHREN ZUM BETREIBEN EINER BIOPROZESSANLAGE ZUR HERSTELLUNG EINES BIOPRODUKTS
PROCÉDÉ DE FONCTIONNEMENT D'UNE INSTALLATION DE BIOPROCÉDÉ POUR LA PRODUCTION D'UN BIOPRODUIT

(43) Date of publication of application: 20.03.2024
(73) Proprietor: Sartorius Stedim Biotech GmbH, 37079 Göttingen (DE)
(72) Inventor: Reger, Lucas Nik, 37073 Göttingen (DE); Saballus, Martin, 37249 Neu-Eichenberg (DE); Kampmann, Markus, 44149 Dortmund (DE); Niemann, Julia, 30171 Hannover (DE)
(74) Representative: Novagraaf International SA

(56) References cited:
- WO-A1-2016/064846
- WO-A1-99/33955
- WO-A2-2011/119826
- CN-A- 103 627 620
- US-A1- 2015 175 950
- US-A1- 2018 094 195

## Description

The present invention relates to a method for operating a bioprocess installation for production of a bioproduct according to claim 1.

The term "bioprocess" presently represents any kind of biotechnological processes, in particular biopharmaceutical processes. Hence, the proposed method may be applied in various fields of biotechnology and for different kinds of bioprocesses. An example of a bioprocess is the use of a bioreactor to cultivate microorganisms like bacterial or mammalian cells under given conditions to produce a bioproduct. Typically, a cell broth is transferred from the bioreactor to a downstream process to separate the bioproduct from the cells and to purify the bioproduct.

The term "bioproduct" in general refers to a product produced by the cells cultivated. Cultivation of cells may currently be used for the production of biopharmaceuticals, in particular proteins, such as human insulin, growth factors, hormones or vaccine proteins, in particular antibodies, antibody derivates, exosomes or the like. The bioproduct may as well be from a group of non-biopharmaceuticals, such as enzymes for food processing, laundry detergent enzymes, biodegradable plastics or biofuels. Additionally or alternatively, cells may also be cultured to produce viral vectors, including lentiviral vectors or the like that are purified and used for applications in the emerging field of cell and gene therapy.

The focus of the present invention is on biopharmaceuticals, more particularly antibodies, and viral vectors that are secreted by the cells into the culture medium.

The term "cells" preferably represents mammalian cells, including HEK293 cells, CHO cells or the like. The cells have preferably been genetically modified, preferably by transfection, to produce the desired bioproduct. Preferably, the bioproduct is secreted from the cells into the culture medium. Alternatively, the bioproduct stays within the cells.

The method in question for operating a bioprocess installation for production of a bioproduct may be applied in various fields of biotechnology and for different kinds of bioprocesses. Exemplarily, the method may be applied to produce a monoclonal antibody using a mammalian CHO suspension cell line.

When operating a bioprocess installation for the production of a bioproduct, process efficiency is particularly important. In particular, process efficiency relates to the cost-efficiency and the footprint of the process. High cost-efficiency and increasing flexibility are driven by the increasing demand for biopharmaceutical drugs, including monoclonal antibodies, vaccines and the like. Another reason for a cost-effective process design is that the material costs associated with the mentioned type of bioprocesses are typically high. A reason for this is that the sterility during the process has to be kept. As a consequence, the bioprocesses typically involve the use of single-use materials and/or cost-intensive sterilization procedures like heat sterilization or the like.

Cost-efficiency is not only related to material costs, but also to an efficient process design. Typically, to get a receptacle, in particular a bioreactor, to a state where it produces the bioproduct, a seed train involving multiple cultivation steps at increasing cultivation volumes needs to be performed. This seed train cultivation takes substantial time, because the cells need to be grown to a certain cell density in each step of the seed train to inoculate the next step and cell growth rates might be comparably low. Additionally, and depending on the final production scale, a substantial number of seed train bioreactors may be required that each need to be run consecutively. Consequently, there is a need to increase process efficiency.

Further, it is desirable that the equipment utilization be as high as possible, meaning that especially the receptacles involved in the method are utilized to a high degree. Additionally, the process design in terms of equipment needed should be as simple as possible to keep the complexity as low as possible.

Document WO 99/33955 A1 relates to the cultivation of preferably anchorage-dependent cells and discloses a method for operating a bioprocess installation for production of a bioproduct, wherein the bioprocess installation comprises a source receptacle for cell cultivation and a harvest receptacle for bioproduction. It has been realized that a fraction of the cells from the source receptacle might be reused as a restart fraction while another fraction of the cells is used for bioproduction. However, there is still a need to improve the production efficiency.

Document WO 2020/010080 A1 relates to a method for culturing mammalian cells in a source receptacle and strongly increasing the cell concentration by adjusting the cultivation medium before transferring it into a harvest receptacle. This is used to achieve cell densities as in perfusion culture without using perfusion culture.

It is a challenge to improve on the known prior art to achieve higher process efficiency.

The invention is based on the problem of improving the known method such that a further optimization regarding the named challenge is reached.

The above-noted object is solved by the features of claim 1.

The main realization of the present invention is that operating a source receptacle in a cyclical production mode to produce cells and operating a harvest receptacle in a non-cyclical production to produce a bioproduct enables an efficient supply of cells to the harvest receptacle and allows a separate optimization of both process steps, including process parameters like initial viable cell concentration and culture medium chosen.

The source receptacle is used to cultivate cells and a fraction of those cells is transferred to a harvest receptacle, where the cells mainly produce bioproduct. Between these steps the cells are centrifuged, allowing to harvest the bioproduct produced in the source receptacle as a collateral, further allowing the increase of the viable cell concentration, allowing to remove potentially toxic byproducts and allowing to change the process conditions between the receptacles. In particular, there may be different optimal process conditions for the production of cells and bioproduct.

By using separate receptacles, the cell production in the source receptacle is particularly simple, while a non-cyclical operation of the harvest receptacle enables adjusting the process conditions in the harvest receptacle to favor bioproduction without having to keep the cells viable. Further, by using the first receptacle for repeated cell production, time, labour- and cost-intensive seed train cultivation may be greatly reduced.

By including a clarification setup with a centrifuge, the process conditions between the source and the harvest receptacle may be adjusted in a particularly simple manner as the cells may easily be washed before being transferred to the harvest receptacle. This way, dead cells and cell debris may also be at least partially removed from the cell broth, which results in an increase in culture viability. Further, washing enables potentially cell-growth inhibiting compounds, e.g., byproducts like lactate and/or ammonium, to be removed or at least reduced from the culture medium. This may enhance the subsequent cultivation in the harvest receptacle and/or simplify the further purification of the bioproduct.

Further, utilization of a centrifuge enables an easy separation of the bioproduct and the cells so that the bioproduct may also be recovered during transfer from the source receptacle to the harvest receptacle.

By operating the source receptacle in a cyclical production mode and the harvest receptacle in a non-cyclical production mode, the process costs and the footprint may be reduced as no large media storage is required and no means for cell retention need to be provided by either receptacle, in particular compared to a perfusion-based process. The utilization of two separate receptacles further provides the option to adapt the cultivation times in each receptacle to each other so that the equipment utilization rate is increased. This further enhances the process efficiency. By using a restart fraction of cells to start the next cultivation in the source receptacle, the downtime of the source receptacle is largely reduced as the source receptacle might be used directly for the next production cycle and does not have to be cleaned.

Proposed is a method for operating a bioprocess installation for production of a bioproduct, wherein the bioprocess installation comprises a source receptacle for cell cultivation, a harvest receptacle for bioproduction and a clarification setup with a centrifuge, wherein the source receptacle is operated in a cyclical production mode, wherein the cyclical production mode comprises, in this order, the steps of:
a) starting the cyclical production mode in the source receptacle with initial cells and cultivation medium,
b) cultivating the cells in the source receptacle, thereby obtaining a cell broth comprising cultivated cells, whereby the source receptacle and the cultivation medium are configured to provide source culture environment conditions for the cultivation of the cells,
c) discharging a discharge fraction of the cell broth from the source receptacle,
d) combining a restart fraction of the cell broth with fresh cultivation medium and repeating step b),
e) repeating steps c) and d) at least once and/or
f) discharging the cell broth obtained from step d) from the source receptacle stopping the cyclical production mode, obtaining a discharge fraction,

wherein the method further comprises the steps of:
   i) transferring the discharge fraction to the clarification setup and centrifuging the discharge fraction via the centrifuge, thereby separating the discharge fraction into at least a centrifuged discharge fraction, supernatant and preferably bioproduct,
   ii) transferring at least part of the centrifuged discharge fraction from step i) and fresh cultivation medium into the harvest receptacle and operating the harvest receptacle in a production mode, whereby the harvest receptacle and the cultivation medium are configured to provide harvest culture environment conditions, producing the bioproduct in the harvest receptacle,
wherein steps i) and ii) are executed at least twice with discharge fractions from an execution of step c) and/or step f).

The term "cell broth" means a suspension of solid cells and possibly cell debris in a cultivation medium and describes the entirety of the cultivation medium and the respective cells cultured in the cultivation medium.

According to claim 2, the cultivation in the source receptacle and the cultivation in the harvest receptacle are carried out in parallel for a substantial amount of time. This way, the process efficiency is drastically enhanced as the equipment utilization is increased with the receptacles being utilized to a large extent.

Claim 3 is directed towards the synchronization between the cultivation time in the source receptacle and the production time in the harvest receptacle. Especially when the number of available receptacle slots is limited, a synchronization between the cultivation time and the production time is important to keep the process as efficient as possible. By synchronizing the cultivation times, extensive downtimes of either receptacle may be reduced, which increases the equipment utilization rate and therefore the process efficiency.

By repeating the cyclical production mode without breaking the synchronicity between the source receptacle slot and the harvest receptacle slot (claim 4), the duration of the seed train is significantly shortened as not all steps of the seed train need to be repeated for each consecutive transfer of cells to the harvest receptacle. Further, each subsequent cyclical production in the source receptacle may be performed at the same source receptacle slot, which reduces the overall footprint of the process as no additional receptacle slots are required. Again, this also increases the equipment utilization rate as not only the receptacles themselves, but also the infrastructure at the respective receptacle slot is utilized as far as possible.

According to claim 5, the harvest fraction from the harvest receptacle is subjected to the clarification setup after producing the bioproduct. Here, the use of a centrifuge in the clarification setup is particularly advantageous as it allows the discharging from the source and harvest receptacles to be carried out process- and equipment-wise similarly, leading to easier work steps and by using the same centrifuge also leading to higher equipment usage time.

Using a sensor arrangement to measure at least one parameter of the source receptacle and/or the harvest receptacle (claim 6) enables to determine the remaining cultivation time and/or the remaining production time. This allows adapting some process step times, in particular the duration of step b), and thereby keeping the synchronization between the cultivation times in the source receptacle and in the harvest receptacle even when unexpected changes occur.

Providing a source electronic process control to control the source culture environment conditions as proposed in claim 7 is advantageous as the source receptacle can be controlled to change its culturing conditions, cell growth rate and the like if necessary. For example, if the harvest receptacle is to be used for production for a longer than planned time, the source receptacle may be adapted to decrease the cell growth rate. Thereby, a target number of cells is reached later, synchronizing the source receptacle and the harvest receptacle instead of having to throw away part of the cell broth from the source receptacle if it reaches its target cell amount too early or instead of risking that the cells lose their viability and may not be used anymore. Further, if bioproduction already occurs in the source receptacle, the amount of bioproduct in the source receptacle might well be increased, as the adaptation of the source receptacle might result in starting and/or increasing bioproduction. Nevertheless, producing a target amount of cells and maintaining a certain viability of the cells remains the priority within the source receptacle.

By predicting the time when a termination condition is reached in the harvest receptacle and adjusting the cultivation conditions in the source receptacle based on this prediction (claim 8), the process flexibility is largely increased as the production can be continued in the harvest receptacle and cell growth in the source receptacle can be adjusted accordingly. This way, the production time in the harvest receptacle can be changed without having to repeat the complete seed train. In particular, the source receptacle may not be optimized for maximum cell growth but instead still have some room to increase, and preferably also decrease, cell growth, thereby providing a control reserve and producing more bioproduct in the source receptacle. Providing a control reserve is a particularly interesting possibility that the proposed method provides. Previously, a harvest receptacle would usually be timed onto a seed train, not allowing greater deviations in the harvest receptacle runtime, even when the cells might still have a high viability and a good production rate. By being able to use the cells for a longer time without desyn-chronizing the seed train, waste and cost per product are reduced.

Claim 9 details possible options to synchronize the source receptacle and the harvest receptacle. To reach a pre-defined cell quantity in the source receptacle needed for subsequent production of the bioproduct in the harvest receptacle, a synchronization strategy may be implemented.

By increasing the cell concentration between step b) and step ii), the subsequent production of the bioproduct in step ii) may be accelerated (claim 10). Increasing the viable cell concentration by centrifugation is particularly advantageous, as not only the cells may be separated from the bioproduct already produced, but the cells may also be washed and/or transferred into another cultivation medium.

By changing at least one process parameter of the source culture environment conditions compared to the harvest culture environment conditions, cell cultivation and bioproduction can be efficiently split between the receptacles (claim 11). In the harvest receptacle, the cell viability does not need to be kept above a certain threshold as the cells can be discharged at the end of the production. Using a centrifuge further makes it a lot easier to change the culture environment between cultivation of the cells and production of the bioproduct.

In a preferred embodiment according to claim 12, the source culture environment and/or the harvest culture environment may be, at least initially, identical for multiple repetitions of steps b) and ii), making the overall method a repetition of identical parallel cultivation and production cycles.

By using batch and/or fed-batch cultivation conditions (claim 13), the overall setup is simplified, especially in comparison to perfusion-based systems as no means for retaining the cells within either receptacle during cultivation are required. This improves the cost-efficiency of the overall process and simplifies operating the bioprocess installation as process complexity is reduced. Further, no large storage space is required to continuously supply the needed amount of culture medium. In contrast to perfusion cultivation the contamination risk, the footprint in form of storage and waste of cultivation medium and the complexity are reduced.

In claim 14, the properties of the centrifuge as well as the cell washing step and cell discharging step are specified. These specifications allow for an optimized preparation of the cells during forward or backward operation of the centrifuge, as well as for optimized conditions for the subsequent bioproduction. The specified centrifuge is particularly well suited for the proposed method.

By transfecting the cells after step c), bioproduction may only be enabled after the cells have been discharged from the source receptacle. This way, no bioproduction occurs in the source receptacle, which is a particularly efficient option to separate the bioproduction from cell growth (claim 15).

The term "transfection" means that a transfer of genetic material is carried out to genetically modify the cells in such a way that the cells produce the desired bioproduct. Cell transfection may be carried out in different ways, including techniques such as electroporation, treatment with chemicals like calcium phosphate, microinjection or the like. Preferably, and as will be explained later, transfection is performed by treatment with chemicals. Alternatively, transfection may also be carried out using viruses or viral vectors.

In the following, an embodiment of the invention is explained with respect to the drawing. The drawing shows in
- Fig. 1,: a bioprocess installation to perform the proposed method for operating a bioprocess installation according to an embodiment of the present invention,
- Fig. 2,: steps a) - e) of the proposed method for operating a bioprocess installation,
- Fig. 3,: steps e) - iii) of the proposed method for operating a bioprocess installation,
- Fig. 4,: an example for synchronizing cultivation in the source receptacle and the harvest receptacle, respectively and
- Fig. 5,: a source receptacle in a source receptacle slot, a harvest receptacle in a harvest receptacle slot and a clarification setup in an exemplary embodiment of the present application.

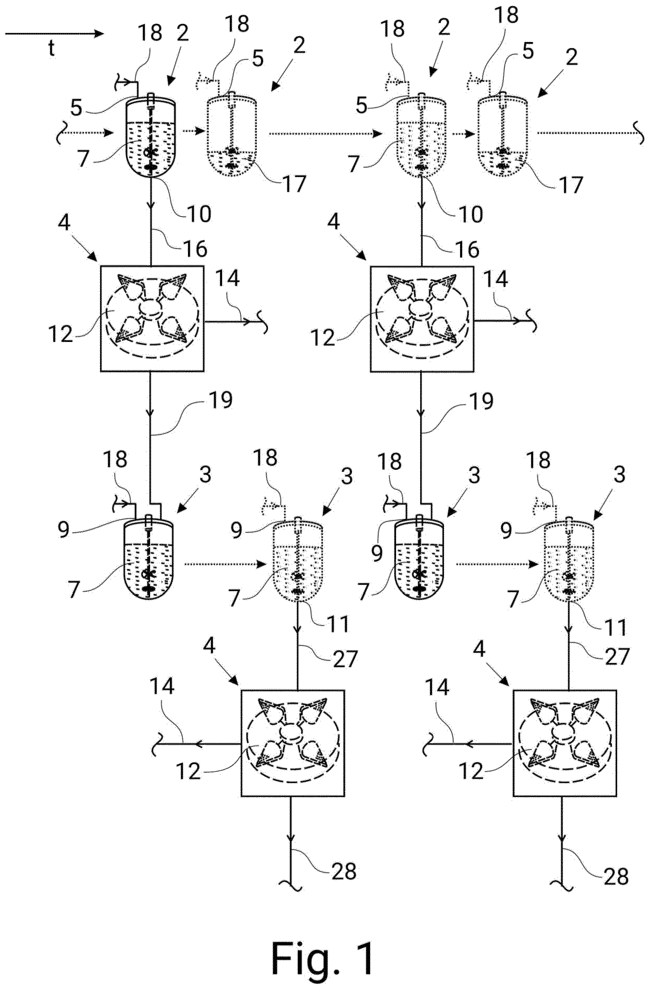

The proposed method for operating a bioprocess installation 1 is depicted in fig. 1. Fig. 1 depicts two cycles of the preferred embodiment of the method whereby fig. 1 schematically shows the method over time from left to right. The bioprocess installation 1 comprises a source receptacle 2 for cell cultivation, a harvest receptacle 3 for bioproduction and at least one clarification setup 4.

In a preferred embodiment and as depicted in fig. 1, the source receptacle 2 is a bioreactor. It is further preferred that a material of the source receptacle 2 is a single-use material. Alternatively, the source receptacle 2 may be a stainless-steel tank bioreactor. The source receptacle 2 here and preferably comprises at least one source receptacle inlet port 5 for the addition of liquids. Each source receptacle inlet port 5 may be connected to a respective liquid line providing the liquid.

The term "liquid" refers to any process liquid handled in the proposed bioprocess installation 1. This includes reagents added to control the source culture environment conditions and/or harvest culture environment conditions, e.g., acid or base to control the pH value, but also washing solutions like buffers used. In general, the term liquids may also refer to culture medium and/or feed medium 6 and/or the cell broth 7.

The term "cultivation medium" relates to the fact that the cells used for the bioprocess grow in or on specially designed solid, semi-solid or liquid cultivation medium, which supply the nutrients required by the respective organisms or cells. A variety of media exist, but usually contain at least a carbon source, a nitrogen source, water, salts, and micronutrients. Here, it is preferred that a chemically-defined cultivation medium 8 is used, meaning that all components of the medium and their respective concentrations are known. However, it is also well possible that a chemically-undefined medium is used, which may contain unknown nutrients and/or nutrients in unknown amounts. A typically example for a chemically undefined medium is a medium that contains FBS (fetal bovine serum).

As noted above, the bioprocess installation 1 further comprises a harvest receptacle 3. In a preferred embodiment and as depicted in fig. 1, the harvest receptacle 3 is a bioreactor. It is further preferred that the material of the harvest receptacle 3 is a single-use material. Alternatively, the harvest receptacle 3 may be a stainless-steel tank bioreactor. Preferably, the harvest receptacle 3 further comprises at least one harvest receptacle inlet port 9 for the addition of liquids. Each harvest receptacle inlet port 9 may be connected to a respective liquid line providing the liquid.

In a preferred embodiment and as depicted in fig. 1, different harvest receptacles 3 are used throughout the method, which will be further explained below.

As depicted in fig. 1, the source receptacle 2 and/or the harvest receptacle 3 preferably comprises at least one source receptacle outlet port 10 and/or at least one harvest receptacle outlet port 11. Preferably, the respective outlet ports are used to withdraw a liquid from the source receptacle 2 and/or the harvest receptacle 3. It is further preferred, that cell broth 7 is withdrawn from the source receptacle 2 and/or the harvest receptacle 3.

In a preferred embodiment, the source receptacle 2 and/or the harvest receptacle 3 are designed to receive a liquid volume of more than 5 liters, preferably more than 15 liters, more preferably more than 50 liters. Preferably, the source receptacle 2 and the harvest receptacle 3 are designed to receive the same maximum liquid volume. It is further preferred, that the source receptacle 2 and the harvest receptacle 3 are of the same geometry. Preferably, also the layout and number of inlet ports and/or outlet ports is identical in the source receptacle 2 and in the harvest receptacle 3. This makes operation of both receptacles simpler.

However, it is also well possible that the source receptacle 2 and the harvest receptacle 3 are different in at least one or all aspects mentioned above. For example, the maximum liquid volume of the harvest receptacle 3 may be lower than the maximum liquid volume of the source receptacle 2.

As also mentioned above, the bioprocess installation 1 further comprises a clarification setup 4. Here and preferably, this clarification setup 4 carries out a physical process using centrifugal force to remove suspended solids, preferably cells, from a liquid phase. In general, the proposed clarification setup 4 can be used to separate any solid/liquid components from each other, including but not limited to cells and media. Preferably, the clarification setup 4 comprises a centrifuge 12 for the clarification of the cell broth 7 by centrifugation. "Centrifugation" is a term for sedimentation of particles in an artificially, by centrifugal forces created, gravitational field, wherein a significant reduction of separation time is achieved via large accelerating forces.

For performing the proposed method, it is preferred and also indicated in fig. 1, that the source receptacle 2 and the centrifuge 12 may be brought into a fluidic coupling. It is further preferred that the centrifuge 12 and the harvest receptacle 3 may be brought into a fluidic coupling as well.

The term "fluidic coupling" means that a fluidic connection is established that may be used to transfer a fluid, preferably a liquid, more preferably at least part of the cell broth 7, from the source receptacle 2 to the centrifuge 12 and/or from the harvest receptacle 3 to the centrifuge 12 and/or to transfer a fluid, preferably a liquid, more preferably the centrifuged 12 cell broth 7 from the centrifuge 12 to the harvest receptacle 3 and/or to a waste 13 location and/or to transfer the supernatant 14 containing the bioproduct from the centrifuge 12 to further purification steps 29.

The centrifuge 12 may be operated in a forward operation and/or in a backward operation and comprises a liquid network of interconnected liquid lines to establish different fluidic connections to and from the centrifuge 12.

In detail proposed is a method for operating a bioprocess installation 1 for production of a bioproduct, wherein the bioprocess installation 1 comprises a source receptacle 2 for cell cultivation, a harvest receptacle 3 for bioproduction and a clarification setup 4 with a centrifuge 12.

Proposed is, that the source receptacle 2 is operated in a cyclical production mode. The cyclical production mode comprises, in this order, the steps of:
a) starting the cyclical production mode in the source receptacle 2 with initial cells 15 and cultivation medium 8,
b) cultivating the cells in the source receptacle 2, thereby obtaining a cell broth 7 comprising cultivated cells, whereby the source receptacle 2 and the cultivation medium 8 are configured to provide source culture environment conditions for the cultivation of the cells,
c) discharging a discharge fraction 16 of the cell broth 7 from the source receptacle 2,
d) combining a restart fraction 17 of the cell broth 7 with fresh cultivation medium 8 and repeating step b),
e) repeating steps c) and d) at least once and/or
f) discharging the cell broth 7 obtained from step d) from the source receptacle 2 stopping the cyclical production mode, obtaining a discharge fraction 16.

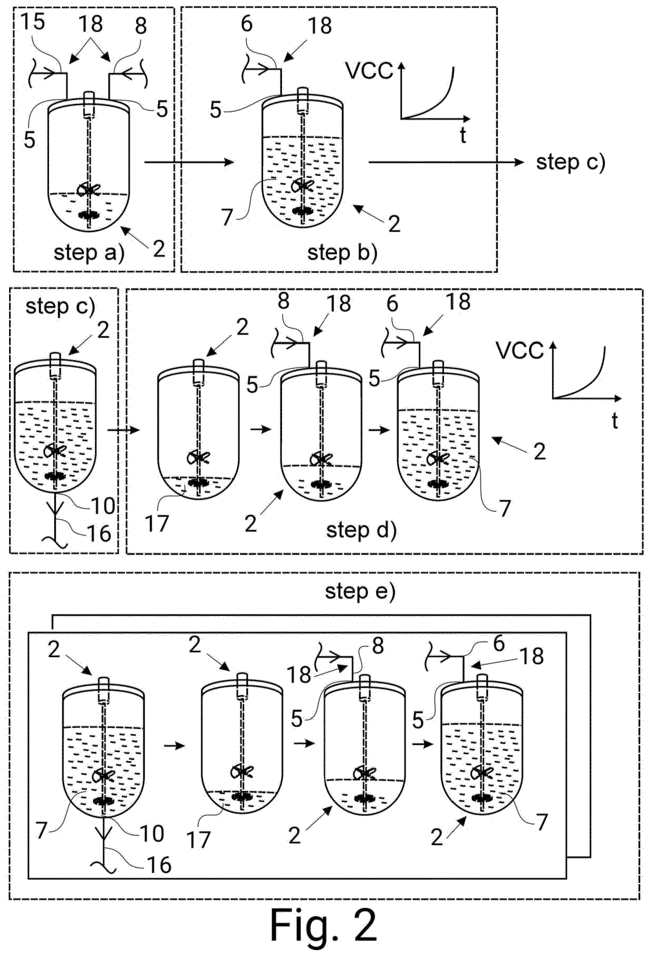

Fig. 1 shows most of the whole method and fig. 2 shows steps a) to e) named respectively. Steps a) to f) relate to the source receptacle 2, the harvest receptacle 3 will be further explained below. The source receptacle 2 is run in a cyclical production mode to cultivate cells. In step a), the cultivation of cells starts by adding initial cells 15 into the source receptacle 2 as depicted in fig. 2 on the top left. Fig. 2 shows how initial cells 15 and cultivation medium 8 are combined in the source receptacle 2 in the usual manner to cultivate the initial cells 15 and increase the absolute number of cells in the source receptacle 2. The initial cells 15 may be the result of a seed train which the cultivation in the source receptacle 2 may be the last step of. Preferably, the initial concentration of viable cells in the source receptacle 2 is adjusted to a predefined value. Preferably, this adjustment is performed by adding cultivation medium 8 until the predefined cell concentration is reached.

Step b) comprises the cultivation. The source receptacle 2 and the cultivation medium 8 together provide, in particular pre-defined, source culture environment conditions. Culture environment conditions comprise all relevant parameters for the cultivation of cells and the production of bioproduct like temperature, stirring speed, pH value, nutrient concentration, etc. The culture environment conditions, partially, change over time. Starting with initial culture environment conditions, the cells consume nutrients and secrete byproducts. Depending on the culture environment conditions and the cells, bioproduct may also be produced to some extent during cultivation in the source receptacle 2. This will be explained further below. Here and preferably the initial source culture environment conditions and/or yet to be explained initial harvest culture environment conditions are pre-defined, meaning there is a plan for the initial source and/or harvest culture environment conditions. Preferably, the later culture environment conditions and therefore all culture environment conditions, for the source and/or harvest receptacle 3, are pre-defined. However, that does not necessarily mean that every parameter is perfectly controlled. Preferably, the source culture environment conditions are adjusted to provide conditions that lead to an optimized cell growth and/or a high overall viability of the cells.

To maintain optimum cell growth conditions and to keep the viability of the cells above a predefined value, it may be required to feed a feed medium 6 during the cultivation of the cells in step b), because certain media components (e.g., a carbon source like glucose) may be depleted from the cultivation medium 8 during the cultivation as they are entirely consumed by the cells. However, depending on the culture behavior and the cultivation time, feeding cultivation medium 8 is only optional and may not be required in any case.

Here it should be noted that the feed medium 6 might be equal to the initial cultivation medium 8. Alternatively, and in a preferred embodiment, the feed medium 6 differs in at least one type and/or at least one concentration of a component from the cultivation medium 8.

Preferably, at least one of the source receptacle inlet ports 5 is a feeding port with a feed line 18 for a controlled feeding of cultivation medium 8 during cultivation according to a predefined feeding profile. Such feeding profiles can be designed as pulse or continuous or a mixed feeding profile, wherein preferably the feeding profile is individually controllable and adjustable. It should be noted that feeding of the cultivation medium 8 results in an increase of the culture broth volume in the source receptacle 2 that depends on the feeding profile and the feed medium 6.

Here and preferably, after the cultivation of cells in step b) has reached a pre-determined termination condition, preferably a target absolute number of cells or a target cell concentration, step b) is terminated and step c) is started. Here and preferably, step b) is focused on providing a fast increase in the number of cells while keeping the cells viable instead of producing bioproduct. Bioproduct may or may not be produced in step b). The bioproduct produced in step b), if any, may have been secreted into the cell broth 7 or may be located inside the cells. In the former case, the bioproduct may be harvested as will be explained later, in the latter case, the bioproduct is preferably not harvested until after the cells have been used to produce bioproduct in the harvest receptacle 3 as will be explained.

In step c), a discharge fraction 16 of the cell broth 7 is removed from the source receptacle 2. This is shown in fig. 1 moving downwards from the source receptacle 2. Preferably and as will be further detailed below, the cell broth 7 is not completely discharged from the source receptacle 2, but a restart fraction 17 is kept in the source receptacle 2. This restart fraction 17 is, in step d), combined with fresh cultivation medium 8 to again cultivate cells in a repetition of step b).

This, at least one, repetition of step b) makes the cultivation in the source receptacle 2 cyclical. Instead of using all cell broth 7 to produce bioproduct, the beginning of the next seed train is skipped, and the restart fraction 17 is used to again cultivate cells in the same source receptacle 2. Here and preferably, the restart fraction 17 stays in the source receptacle 2 and is not temporarily removed from the source receptacle 2 between repetitions of step b). That way the source receptacle 2 can be used for more than one cycle, without changing or cleaning the source receptacle 2. This is indicated by the dashed lines used for the source receptacle 2 in fig. 1. The repetition of step b) is shown when moving at the top of fig. 1 to the right and/or as part of step d) depicted in fig. 2.

The repetition of step b), preferably each repetition of step b), may be identical to the first execution of step b) in some or all aspects, in particular regarding the source environment conditions or some of the source environment conditions. All aspects described herein with regard to step b) may be identical for at least one, preferably all executions of step b), alone or in any combination.

The restart fraction 17 and the discharge fraction 16 preferably sum up to at least 95 %, preferably at least 99 %, more preferably 100 % of the cell broth 7 in the source receptacle 2 when starting step c) or ending step b).

Preferably, the discharge fraction 16 is at least 50 %, preferably at least 70 %, more preferably at least 90 % of the volume of the cell broth 7 at the beginning of step c) or at the end of step b). Preferably, the cell broth 7 is discharged from the source receptacle 2 using the source receptacle outlet port 10. As will be explained in detail below, it is preferred that the cell broth 7 is transferred to the centrifuge 12.

In step e), steps c) and d) may be repeated for at least one, preferably at least two, more preferably at least three times. For each repetition, the discharge fraction 16 is transferred to a harvest receptacle 3 as will be explained. Therefore, the source receptacle 2 cyclically produces cell broth 7 for bioproduct production without needing a seed train each time.

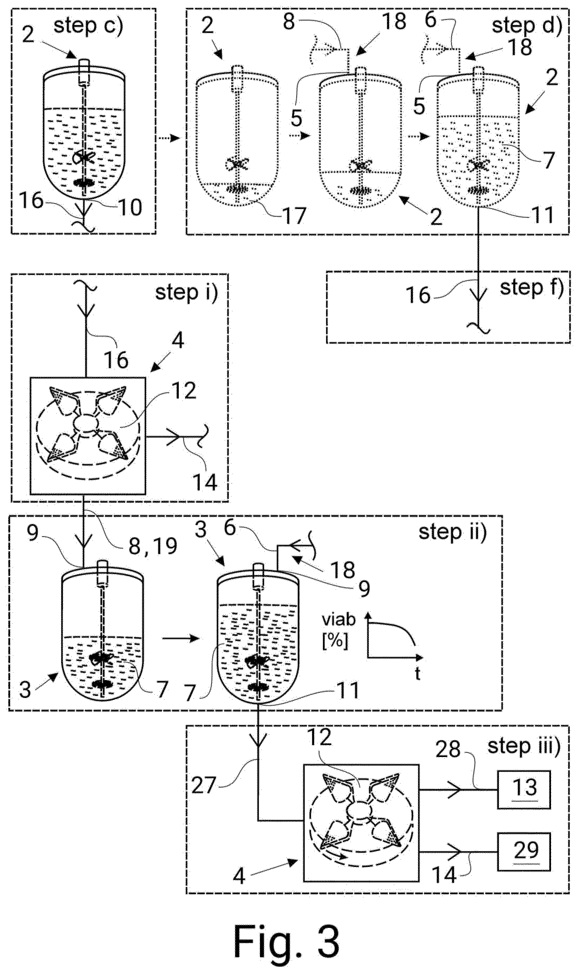

In step f), which is depicted in fig. 3, the cyclical production is stopped. A discharge fraction 16 which preferably is at least 95 %, preferably at least 99 %, more preferably 100 % of the cell broth 7 in the source receptacle 2, is, here and preferably, again transferred to a harvest receptacle 3 as will be explained. Fig. 2 shows steps a) to e). Fig. 2 is to be read from left to right in each of the three rows. VCC is the viable cell concentration in the source receptacle 2 which increases and decreases with the different method steps. The volume in the source receptacle 2 may (as shown) or may not change during the steps, depending on the specifics of the process.

The discharge fraction 16, whether from step d) or step f), is not directly transferred to the harvest receptacle 3, but submitted to a clarification setup 4.

Turning now to the harvest receptacle 3 side and the transfer to the harvest receptacle 3 shown in fig. 1 below the first row and in fig. 3, the proposed method further comprises the steps of:
i) transferring the discharge fraction 16, resulting either from step c) or f), to the clarification setup 4 and centrifuging the discharge fraction 16 via the centrifuge 12, thereby separating the discharge fraction 16 into at least a centrifuged discharge fraction 19 and supernatant 14 and preferably bioproduct,
ii) transferring at least part of the centrifuged discharge fraction 19 from step i) and fresh cultivation medium 8 into the harvest receptacle 3 and operating the harvest receptacle 3 in a production mode, here and preferably a non-cyclical production mode, whereby the harvest receptacle 3 and the cultivation medium 8 are configured to provide harvest culture environment conditions, producing the bioproduct in the harvest receptacle 3,
wherein steps i) and ii) are executed at least twice with discharge fractions 16 from an execution of step c) and/or step f).

Step i) relates to the centrifugation of the discharge fraction 16 via the centrifuge 12, preferably always the same centrifuge 12. This centrifugation can be used to achieve several positive effects.

The discharge fraction 16 can be transferred to the centrifuge 12 via a multi-use or temporary liquid line. Multi-use means that the liquid line is used for at least two discharge fractions 16. A pumping arrangement may be used to transfer the discharge fraction 16. The discharge fraction 16 is then centrifuged 12. This centrifugation may increase the cell concentration such that a higher initial cell concentration may be used in step ii). The cells may be washed, removing cell debris and/or accumulated byproducts or the like. Preferably bioproduct is separated from the discharge fraction 16, in particular if the bioproduct was secreted into the cell broth 7. The bioproduct may be separated directly by the centrifuge during the centrifugation which produces the centrifuged discharge fraction 16 or may be contained in the supernatant 14 and separated in a later or earlier step. The centrifugation in particular allows changing the culture environment conditions between the source receptacle 2 and the harvest receptacle 3 as supernatant 14 is removed and can be substituted by fresh cultivation medium 8 with different properties. Removing accumulated byproducts further increases the efficiency of the following bioproduction.

At least part of the centrifuged discharge fraction 19, preferably, at least 95 %, more preferably at least 99 %, more preferably 100%, of the centrifuged discharge fraction 19, are preferably combined in step i) with fresh cultivation medium 8 and then used in step ii) to produce bioproduct. Preferably, the cells are combined with the fresh cultivation medium 8 during the centrifugation process and this centrifuged discharge fraction is then transferred to the harvest receptacle 3. The fresh cultivation medium 8 may have a volume of less than 90% of the volume of the discharge fraction 16, preferably less than 70%, more preferably less than 50% of the volume of the discharge fraction 16. Here it is preferred that the cultivation medium 8 of the centrifuged discharge fraction 19 differs in at least one type and/or amount of nutrient from the cultivation medium used in step a) and/or the feed medium used in step b).

Preferably, the harvest culture environment conditions in the harvest receptacle 3 are substantially different from the source culture environment conditions. The former may be optimized for bioproduct production and/or the latter for cell growth. Exemplarily, a feed medium 6 may be supplied via a harvest receptacle inlet port 9. The feed medium 6 and/or the feeding profile may be the same feed medium 6 and/or feeding profile as may be used in step b) in the source receptacle 2. However, it is preferred that the feed medium 6 and/or the feeding profile differs in at least one parameter from the feed medium 6 and/or the feeding profile used in step b) in the source receptacle 2. An example for such a parameter may be the nutrient composition of the feed medium 6.

As depicted in fig. 1, steps i) and ii) are repeated too, however preferably with different, in particular single-use, harvest receptacles 3 or within a harvest receptacle 3 cleaned between the executions. The source receptacle 2 and the harvest receptacle 3 are not the same receptacle.

Here and preferably, the bioproduct is extracted from a cell broth 7 produced in the harvest receptacle 3 after each execution of step ii). As there is preferably no further use of the cells from the harvest receptacle 3, the harvest culture environment conditions may be such that the cells die during the bioproduction decreasing the cell viability to a lower value compared to the cultivation in the source receptacle 2.

The duration of step b) and/or step ii) may be at least one day, preferably at least two days.

Here and preferably the source receptacle 2 and the harvest receptacle 3 are not directly connected process-wise. Here and preferably no cells are transferred from the harvest receptacle 3 to the source receptacle 2.

Looking again at the centrifuge 12, the centrifuge 12 can be operated in a forward operation for cell separation and/or cell washing. "Forward operation" means one out of two possible fluid flow directions of a centrifuge 12 and describes the operation leading to a separation of liquid and solid particles, such as culture medium and cells. The liquid obtained this way is the supernatant 14. This separation allows, on the one hand, a washing of separated cells with a washing buffer, preferably PBS buffer, or media, preferably cultivation medium 8, and/or, on the other hand, to obtain the supernatant 14.

Alternatively, the centrifuge 12 can be operated in a backward operation. "Backward operation" means the second out of two possible fluid flow directions of a centrifuge 12 and describes the operation leading to a discharge of the separated solid particles, preferably cells. The product to be obtained in backward operation can be the centrifuged discharge fraction 19.

According to one embodiment it is proposed, that for at least one, preferably at least two, more preferably each execution of steps b) and ii), the cultivation of cells in step b) and the bioproduction in step ii) are executed at least partially in parallel, preferably, that the executions of steps b) and ii) run for at least 50%, preferably at least 75%, more preferably at least 90%, of the time used for cultivation in step b) and/or the time used for bioproduction in step ii) and/or the time used for execution of step b) and/or step ii) in parallel. The times mentioned are the times from the initial filling of the respective receptacle or, partial, re-filling with fresh cultivation medium 8 until the removal of the discharge fraction 16 or removal of at least an essential part of the cell broth 7 of the harvest receptacle 3 for harvesting the bioproduct. If the bioproduct is harvested from the harvest receptacle 3 and/or the discharge fraction 16 is discharged in more than one fraction, the last fraction counts.

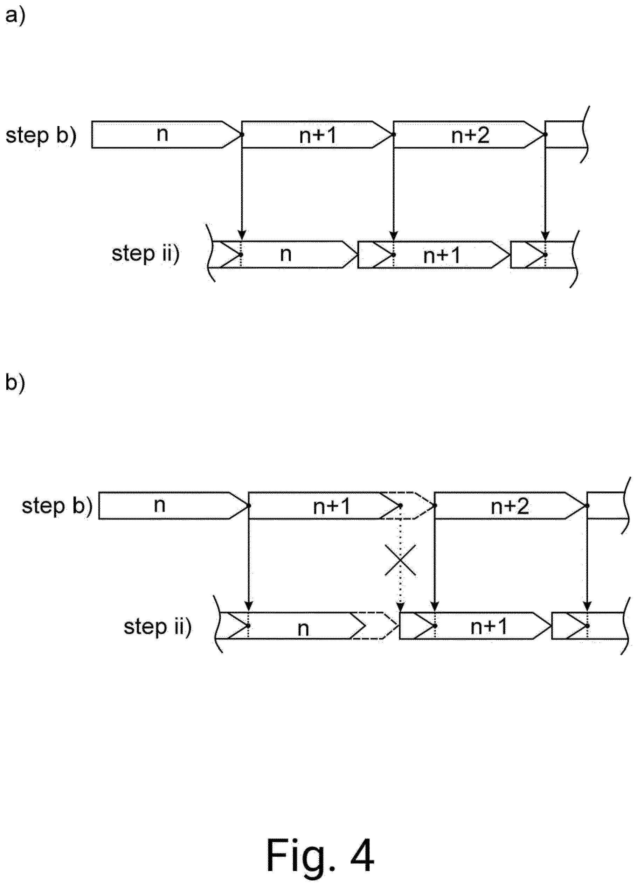

As depicted in fig. 4a, after step b) was carried out for example for the first time (n = 1) as part of the proposed method, a restart fraction 17 of cells may be combined with fresh cultivation medium 8 (step d)) and step b) may be repeated at least a second time (n+1). However, it is preferred that step b) is repeated until the cyclical production mode is stopped by executing step f) of the proposed method.

As soon as steps c) and i) have been carried out on the discharge fraction 16 of step b) for the first time (n), a first execution of step ii) (n) may be carried out in the harvest receptacle 3.

By performing the second execution of step b) (n+1) in parallel to the first execution of step ii) (n), both receptacles operate in parallel. By operating the cultivation in the receptacles in parallel for a predefined amount of time, the utilization of the receptacles and the associated equipment is maximized.

As will be further described below, different strategies may be applied to synchronize the cultivation times in both receptacles.

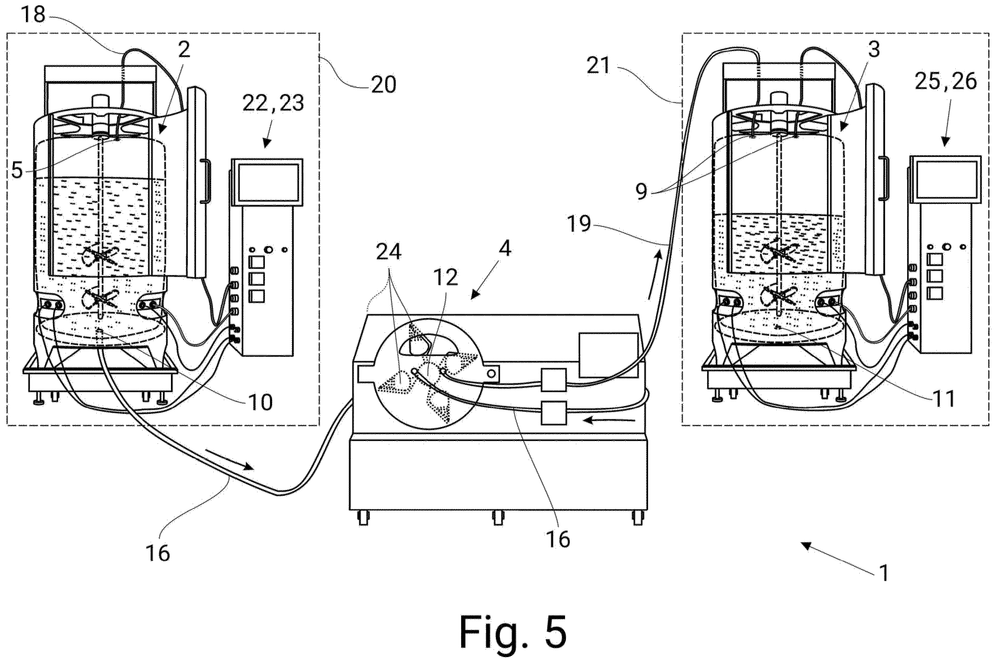

With view towards fig. 5, a bioproduction may be optimized with regard to general process planning by providing slots for the receptacles, planning movement of media and process times and the like. Therefore, it may be the case, that a single source receptacle slot 20 is paired with a single harvest receptacle slot 21, that step b) is executed for a, in particular pre-determined, cultivation time, that step ii) is executed for a, in particular pre-determined, production time, that the cultivation time and the production time and therefore the source receptacle slot 20 and the harvest receptacle slot 21 are synchronized such that for at least two consecutive executions, preferably all executions, of steps ii) the same harvest receptacle slot 21 is used.

One slot comprises exactly one source or harvest receptacle 3, preferably at least or exactly one electronic process control for the receptacle and a defined location.

Here and preferably, after at least one repetition, preferably after each repetition, of steps b), c) and i), the harvest receptacle slot 21 is already prepared to receive the centrifuged discharge fraction 19. When the term "repetition" is used, any execution except the first execution is meant. Therefore, the method is synchronized such that the harvest receptacle 3 is not only finished, but also necessary steps for preparation of the harvest receptacle slot 21, for example connecting a new receptacle, are done. Preferably, the harvest receptacle slot 21 is already prepared when the first fraction of the centrifuged discharge fraction 19 is ready to be discharged from the centrifuge 12 or at least it is prepared so soon that no relevant part of the cells die.

The term "pairing" presently means that the cells cultivated in the source receptacle 2 of the source receptacle slot 20 are transferred to the harvest receptacle 3 located in the harvest receptacle slot 21. The pairing of a source receptacle slot 20 and a harvest receptacle slot 21 is preferably maintained for at least 2 executions of steps b) and ii) and/or for preferably at least 3 weeks, more preferably at least 5 weeks.

As depicted in fig. 5, the source receptacle slot 20 comprises the physical space to receive the source receptacle 2. Preferably, the source receptacle slot 20 comprises the physical place to install the source receptacle 2. As depicted in fig. 5, transport means may be used to transport the source receptacle 2 to the space provided by the source receptacle slot 20. It is further preferred that the source receptacle slot 20 comprises a source electronic process control 22 and that the source electronic process control 22 is integrated into a source electronic process control unit 23. Preferably, the source electronic process control unit 23 is located next to the source receptacle 2.

It is further preferred, that the source electronic process control unit 23 is connected to at least one sensor placed inside or at the source receptacle 2. As depicted in fig. 5, this sensor might be a pH probe or a temperature probe or any other sensor suited to monitor and preferably also control the at least one process parameter.

Preferably, cultivation in the source receptacle slot 20 is carried out for a pre-determined cultivation time. After the predetermined cultivation time, a discharge fraction 16 of the cell broth 7 is discharged from the source receptacle 2 and transferred to the centrifuge 12.

As further depicted in fig. 5, the source receptacle 2 is connected to the clarification setup 4 comprising the centrifuge 12. Here it is to be noted that the complex network of fluid lines that guides the liquids to and from the centrifuge chambers 24 is not depicted to maintain clarity. Preferably, the centrifuge 12 is designed as a fluidized-bed centrifuge 12 comprising at least one centrifuge chamber 24. In a preferred embodiment and as depicted in fig. 5, the centrifuge 12 comprises four centrifuge chambers 24.

It is to be noted here, that the connection between the source receptacle 2 and the centrifuge 12 and/or the harvest receptacle 3 and the centrifuge 12 is preferably only maintained until the respective transfer steps have been carried out. This way, the centrifuge 12 may be utilized elsewhere, when no transfer to and/or from the centrifuge 12 and no centrifugation is required.

As depicted in fig. 5, the harvest receptacle slot 21 comprises the physical space to receive the harvest receptacle 3. Preferably, the harvest receptacle slot 21 comprises the physical place to install the harvest receptacle 3. As depicted in fig. 5, transport means may be used to transport the harvest receptacle 3 to the space provided by the harvest receptacle slot 21. It is further preferred that the harvest receptacle slot 21 comprises a harvest electronic process control 25 and that the harvest electronic process control 25 is integrated into a harvest electronic process control unit 26. Preferably, the harvest electronic process control unit 26 is located next to the harvest receptacle 3.

It is further preferred, that the harvest electronic process control unit 26 is connected to at least one sensor placed inside or at the harvest receptacle 3. As depicted in fig. 5, this sensor might be a pH probe or a temperature probe or any other sensor suited to monitor and preferably also control the at least one process parameter.

As becomes clear from fig. 5, preferably the source receptacle 2 and the harvest receptacle 3 are not directly connected. Further, and as also depicted in fig. 5, once the cell broth 7 has been transferred from the source receptacle 2 to the centrifuge 12, the cell broth 7 is not transferred back to the source receptacle 2. Generally it would be possible to submit the whole cell broth 7 from the source receptacle 2 to the centrifuge 12 and transfer the restart fraction 17 from the centrifuge, directly, or indirectly for example via the harvest receptacle 3, back to the same and not cleaned, sterilized or the like source receptacle 2. Here and preferably however, the restart fraction 17 stays inside the source receptacle 2.

In the preferred embodiment steps a) to f) are synchronized, time-wise, with repeated seed trains such that after step f) step a) is repeated at the same source receptacle slot 20 without breaking synchronicity between the source receptacle slot 20 and the harvest receptacle slot 21. After a number of repetitions of step b), the cells may become genetically unstable. Preferably they are replaced with new initial cells 15 from a seed train without changing the repetitions in the harvest receptacle 3. In that way, production can be maintained forever in theory.

According to one embodiment it is proposed, that the method further comprises the step of
iii) transferring a harvest fraction 27 from the harvest receptacle 3 to the clarification setup 4 and centrifuging the harvest fraction 27 via the centrifuge 12, thereby separating the bioproduct, when located in the supernatant 14 from the harvest fraction 27. Here and preferably, step iii) is executed after each step ii). Preferably, the harvest fraction 27 is the complete cell broth 7 from the harvest receptacle 3. Again, the bioproduct, when located in the supernatant 14, may be forwarded to further purification steps 29 while the harvest fraction 28 is preferably directed to a waste 13 location. Alternatively, when the bioproduct is present within the cells, the harvest fraction 28 may be directed to further purification steps and the supernatant may be directed to a waste location.

In an especially preferred embodiment the same clarification setup 4 is used in step i) and step iii).

According to one embodiment it is proposed, that the bioprocess installation 1 comprises a sensor arrangement, that the sensor arrangement comprises at least one sensor placed in or at the source receptacle 2 and/or at least one sensor placed in or at the harvest receptacle 3, preferably, that at least one sensor is configured to measure at least one process parameter out of the group of oxygen concentration, carbon dioxide concentration, pH, temperature, conductivity, pressure, viable cell concentration, viable cell production rate, product concentration, productivity, stirring speed and/or the culture medium, including the concentration of nutrients like carbon-source concentration, nitrogen-source concentration, amino acid concentration, growth factor concentration or the like for the source receptacle 2 and/or for the harvest receptacle 3. As mentioned, the sensor may be connected to the respective electronic process control unit and used to control the respective culture environment conditions in the source receptacle 2 and/or in the harvest receptacle 3.

The term "process parameter" is to be understood in a broad sense and includes at least one process parameter that may be monitored and preferably also controlled within the source receptacle 2 and/or the harvest receptacle 3.

It should be noted that additional process parameters may be derived from monitoring more than one process parameter. For example, the change in the bioproduct concentration over time, which may also be referred to as productivity, may be derived by relating the bioproduct concentration to the cultivation time. Further, the cell growth rate may be determined from evaluating the change of the viable cell concentration over the cultivation time.

As already mentioned, in may be the case, that the bioprocess installation 1, in particular the source receptacle slot 20, comprises a source electronic process control 22 for controlling the source culture environment conditions, preferably that the source electronic process control 22 is connected to the at least one sensor placed in or at the source receptacle 2.

Further it may be the case, that step ii) is terminated after a pre-determined termination condition is reached, preferably, that at least one sensor measurement related to the termination condition is measured repeatedly, in particular cyclically or continuously, by the sensor placed in or at the harvest receptacle 3 during at least one, in particular each, execution of step ii), more preferably, that the sensor measurement is used, in particular by a harvest electronic process control 25, to predict a time of reaching the termination condition, that the source culture environment conditions in step c) are controlled, in particular by the source electronic process control 22, to synchronize step c) with step ii). The sensors or measured process parameters named above apply.

What's very interesting now is that in some cases, the harvest receptacle 3 may be used for longer or shorter than planned and that may be noticed or deliberately decided only during the respective step ii). Instead of having to interrupt step ii) because step b) is about to finish, step b) may be planned with a control reserve from the beginning. Here and preferably, step b) can be slowed down or sped up. This leads to longer than the theoretical best for the duration of step b), but this change may in particular be used to keep the viability of the cells in the source receptacle 2 above a pre-defined threshold and/or to produce more bioproduct already during step b) and gaining a control reserve to speed up step b). Additionally or alternatively, step b) may be slowed down by changing the source culture environment conditions to produce more bioproduct and/or have less cell growth and thereby for example less toxic byproduct production, which may decrease the overall culture viability.

In general, a synchronization strategy may be executed, in particular applied to the source receptacle 2, to keep the source and harvest receptacles 3 and/or their slots synchronized. Such a synchronization is particularly important, as the cells may not be kept viable within the source receptacle 2 for a prolonged time without adjusting the source culture environment conditions and/or at least one process parameter within the source receptacle 2. The goal is to provide a pre-defined amount of cells in the source receptacle 2 when the harvest receptacle slot 21 has been prepared to execute step ii). For this, the cultivation time in the source receptacle 2 is synchronized with the cultivation time in the harvest receptacle 3.

Such a synchronization strategy is depicted in fig. 4b. For example, a temperature inside the source receptacle 2 may be reduced to prolong the cultivation time in the source receptacle 2. Additionally or alternatively, feed medium may be supplied to the cultivated cells or the supply of feed medium may be continued to prolong the cultivation time in the source receptacle 2.

The respective process parameters may be predicted based on historical data. In case the prediction is not good enough, incremental changes to the process parameters may be made. Another synchronization strategy may include a change in the split of the cell broth 7 from the source receptacle 2 into discharge fraction 16 and restart fraction 17. If more cell broth 7 or a higher viable cell concentration has been reached in a prolonged step b), the restart fraction 17 may be decreased, either absolutely or relatively, to allow another longer step b) and/or step ii).

A pre-determined termination condition for the harvest receptacle 3 may be that at least one process value reaches a certain value, in particular, and as indicated in fig. 3, that the viability (viab) falls below a predetermined threshold, and/or, that a certain cultivation time is reached, and/or, that a certain filling volume is reached, and/or, that, a certain bioproduct concentration is reached, and/or, that a ratio between product and by-product concentration falls below a predetermined threshold, and/or, that a certain amount of byproduct is reached, and/or, that the viable cell concentration reaches a certain value, and/or, that a certain process value was maintained for a certain amount of time.

In order to estimate the time, when the cultivation in the harvest receptacle 3 will reach the pre-defined termination condition, various process parameters with appropriate process models can be used, such as cell viability, cell specific productivity, product concentration, level of impurities (e.g. host cell impurity concentration) or any CQA of the product.

According to one embodiment it is proposed, that if a deviation between the predicted time of reaching the termination condition and a planned end of the production time is detected, in particular by the source electronic process control 22 or the harvest electronic process control 25, a synchronization strategy is executed, preferably, that the synchronization strategy comprises adapting the source culture environment conditions and/or harvest culture environment conditions to reduce the resulting deviation between the source receptacle 2 and the harvest receptacle 3, in particular to reach a pre-defined absolute cell quantity in the source receptacle 2 prior to initiating step c). This may lead to step c) starting earlier or later.

Another synchronization strategy may include changing a composition of the feed medium 6 and/or a point in time of adding the feed medium 6 and/or changing a target filling volume in the source receptacle 2.

According to one embodiment it is proposed, that an initial viable cell concentration in step ii) is higher than a final viable cell concentration in step b), preferably, that the cell concentration is increased by the centrifugation in step i). This may be particularly advantageous also in a case where the source receptacle 2 has a bigger volume than the harvest receptacle 3, for example at least 10% bigger.

**It** may also be the case, that at least one process parameter of the source culture environment conditions, in particular initial source culture environment conditions, in at least one, in particular each, execution of step b) and of the harvest culture environment conditions, in particular initial harvest culture environment conditions, in at least one, in particular each, execution of step ii) is set differently, preferably, that the at least one process parameter is optimized in the source culture environment conditions for cultivation of cells over production of bioproduct and/or that the at least one process parameter is optimized in the harvest culture environment conditions for production of bioproduct over cultivation of cells. Different here means substantially different, not different within tolerances or near tolerances.

According to one embodiment it is proposed, that the initial source culture environment, in particular the source culture environment, and/or the initial harvest culture environment, in particular the harvest culture environment, is essentially identical for at least one, in particular each, repetition of step b) and/or step ii) respectively.

According to one embodiment it is proposed, that cultivating the cells in step b) of the cyclical production mode is performed under batch cultivation conditions or fed-batch cultivation conditions, and/or, that the production mode of step ii) is performed under fed-batch cultivation conditions.

This may be the case at least once, preferably at least twice, more preferably each time the respective step is executed.

According to one embodiment and as already mentioned it is proposed, that the centrifuge 12 is a fluidized bed centrifuge 12, preferably, that the centrifuge 12 is operated in a forward operation for cell separation and/or cell washing and a backward operation for cell discharging, more preferably, that the centrifuge 12 is operated in a backward operation for transferring the centrifuged discharge fraction 19 to the harvest receptacle 3 between steps i) and ii).

According to one embodiment it is proposed, that a transfection step is carried out after step c) on the discharge fraction 16, or, that the bioproduct is produced by the cells in the source receptacle 2.

Here, the use of a centrifuge 12, preferably a fluidized-bed centrifuge, as the clarification setup 4 provides simple means to carry out a transfection step. For the definition of transfection, reference is made to the definition given above. For example, by being able to supply a wash solution and/or fresh culture medium during centrifugation in step i), treatment with calcium phosphate for transfection may easily be carried out as the DNA to be transferred into the cells and the calcium phosphate may be supplied during the centrifugation. Transfection may also be carried out prior to or after centrifugation outside the source receptacle 2.

### Reference numbers

- 1: bioprocess installation
- 2: source receptacle
- 3: harvest receptacle
- 4: clarification setup
- 5: source receptacle inlet port
- 6: feed medium
- 7: cell broth
- 8: cultivation medium
- 9: harvest receptacle inlet port
- 10: source receptacle outlet port
- 11: harvest receptacle outlet port
- 12: centrifuge
- 13: waste
- 14: supernatant
- 15: initial cells
- 16: discharge fraction
- 17: restart fraction
- 18: feed line
- 19: centrifuged discharge fraction
- 20: source receptacle slot
- 21: harvest receptacle slot
- 22: source electronic process control
- 23: source electronic process control unit
- 24: centrifuge chamber
- 25: harvest electronic process control
- 26: harvest electronic process control unit
- 27: harvest fraction
- 28: centrifuged harvest fraction
- 29: purification step

## Claims

1. Method for operating a bioprocess installation (1) for production of a bioproduct, wherein the bioprocess installation (1) comprises a source receptacle (2) for cell cultivation, a harvest receptacle (3) for bioproduction and a clarification setup (4) with a centrifuge (12),
wherein the source receptacle (2) is operated in a cyclical production mode,
wherein the cyclical production mode comprises, in this order, the steps of:
a) starting the cyclical production mode in the source receptacle (2) with initial cells (15) and cultivation medium (8),
b) cultivating the cells in the source receptacle (2), thereby obtaining a cell broth (7) comprising cultivated cells, whereby the source receptacle (2) and the cultivation medium (8) are configured to provide source culture environment conditions for the cultivation of the cells,
c) discharging a discharge fraction (16) of the cell broth (7) from the source receptacle (2),
d) combining a restart fraction (17) of the cell broth (7) with fresh cultivation medium (8) and repeating step b),
e) repeating steps c) and d) at least once and/or
f) discharging the cell broth (7) obtained from step d) from the source receptacle (2) stopping the cyclical production mode, obtaining a discharge fraction (16),
wherein the method further comprises the steps of:
i) transferring the discharge fraction (16) to the clarification setup (4) and centrifuging the discharge fraction (16) via the centrifuge (12), thereby separating the discharge fraction (16) into at least a centrifuged discharge fraction (19) and supernatant (14) and preferably bioproduct,
ii) transferring at least part of the centrifuged discharge fraction (19) from step i) and fresh cultivation medium (8) into the harvest receptacle (3) and operating the harvest receptacle (3) in a production mode, whereby the harvest receptacle (3) and the cultivation medium (8) are configured to provide harvest culture environment conditions, producing the bioproduct in the harvest receptacle (3),
wherein steps i) and ii) are executed at least twice with discharge fractions (16) from an execution of step c) and/or step f).

2. Method according to claim 1, **characterized in that** for at least one, preferably at least two, more preferably each execution of steps b) and ii), the cultivation of cells in step b) and the bioproduction in step ii) are executed at least partially in parallel, preferably, that the executions of steps b) and ii) run for at least 50%, preferably at least 75%, more preferably at least 90%, of the time used for cultivation in step b) and/or the time used for bioproduction in step ii) and/or the time used for execution of step b) and/or step ii) in parallel.

3. Method according to claim 1 or 2, **characterized in that** a single source receptacle slot (20) is paired with a single harvest receptacle slot (21), that step b) is executed for a, in particular pre-determined, cultivation time, that step ii) is executed for a, in particular pre-determined, production time, that the cultivation time and the production time and therefore the source receptacle slot (20) and the harvest receptacle slot (21) are synchronized such that for at least two consecutive executions, preferably all executions, of steps ii) the same harvest receptacle slot (21) is used.

4. Method according to claim 3, **characterized in that** steps a) to f) are synchronized with repeated seed trains such that after step f) step a) is repeated at the same source receptacle slot (20) without breaking synchronicity between the source receptacle slot (20) and the harvest receptacle slot (21).

5. Method according to one of the preceding claims, **characterized in that** the method further comprises the step of
iii) transferring a harvest fraction (27) from the harvest receptacle (3) to the clarification setup (4) and centrifuging the harvest fraction (27) via the centrifuge (12), thereby separating the bioproduct from the harvest fraction (27), preferably, that step iii) is executed after each step ii).

6. Method according to one of the preceding claims, **characterized in that** the bioprocess installation (1) comprises a sensor arrangement, that the sensor arrangement comprises at least one sensor placed in or at the source receptacle (2) and/or at least one sensor placed in or at the harvest receptacle (3), preferably, that at least one sensor is configured to measure at least one process parameter out of the group of carbon-source concentration, nitrogen-source concentration, amino acid concentration, growth factor concentration, oxygen concentration, carbon dioxide concentration, pH, temperature, conductivity, pressure, biomass concentration, biomass production rate, product concentration, productivity, oxygen uptake rate and/or stirring speed for the source receptacle (2) and/or for the harvest receptacle (3).

7. Method according to one of the preceding claims, **characterized in that** the bioprocess installation (1), in particular the source receptacle slot (20), comprises a source electronic process control (22) for controlling the source culture environment conditions, preferably that the source electronic process control (22) is connected to the at least one sensor placed in or at the source receptacle (2).

8. Method according to one of the preceding claims, **characterized in that** step ii) is terminated after a pre-determined termination condition is reached, preferably, that at least one sensor measurement related to the termination condition is measured repeatedly, in particular cyclically or continuously, by the sensor placed in or at the harvest receptacle (3) during at least one, in particular each, execution of step ii), more preferably, that the sensor measurement is used, in particular by a harvest electronic process control (25), to predict a time of reaching the termination condition, that the source culture environment conditions in step b) are controlled, in particular by the source electronic process control (22), to synchronize step b) with step ii).

9. Method according to claims 3 and 8, **characterized in that** if a deviation between the predicted time of reaching the termination condition and a planned end of the production time is detected, in particular by the source electronic process control (22) or the harvest electronic process control (25), a synchronization strategy is executed, preferably, that the synchronization strategy comprises adapting the source culture environment conditions and/or harvest culture environment conditions to reduce the resulting deviation between the source receptacle (2) and the harvest receptacle (3), in particular to reach a pre-defined absolute cell quantity in the source receptacle (2) prior to initiating step c).

10. Method according to one of the preceding claims, **characterized in that** an initial viable cell concentration in step ii) is higher than a final viable cell concentration in step b), preferably, that the cell concentration is increased by the centrifugation in step i).

11. Method according to one of the preceding claims, **characterized in that** at least one process parameter of the source culture environment conditions, in particular initial source culture environment conditions, in at least one, in particular each, execution of step b) and of the harvest culture environment conditions, in particular initial harvest culture environment conditions, in at least one, in particular each, execution of step ii) is set differently, preferably, that the at least one process parameter is optimized in the source culture environment conditions for cultivation of cells over production of bioproduct and/or that the at least one process parameter is optimized in the harvest culture environment conditions for production of bioproduct over cultivation of cells.

12. Method according to one of the preceding claims, **characterized in that** the initial source culture environment, in particular the source culture environment, and/or the initial harvest culture environment, in particular the harvest culture environment, is essentially identical for at least one, in particular each, repetition of step b) and/or step ii) respectively.

13. Method according to one of the preceding claims, **characterized in that** cultivating the cells in step b) of the cyclical production mode is performed under batch cultivation conditions or fed-batch cultivation conditions, and/or, that the production mode of step ii) is performed under fed-batch cultivation conditions.

14. Method according to one of the preceding claims, **characterized in that** the centrifuge (12) is a fluidized bed centrifuge (12), preferably, that the centrifuge (12) is operated in a forward operation for cell separation and/or cell washing and a backward operation for cell discharging, more preferably, that the centrifuge (12) is operated in a backward operation for transferring the centrifuged discharge fraction (19) to the harvest receptacle (3) between steps i) and ii).

15. Method according to one of the preceding claims, **characterized in that** a transfection step is carried out after step c) on the discharge fraction (16), or, that the bioproduct is produced by the cells in the source receptacle (2).

## Patentansprüche

1. Verfahren zum Betreiben einer Bioprozessanlage (1) zur Herstellung eines Bioprodukts, wobei die Bioprozessanlage (1) einen Quellbehälter (2) zur Zellkultivierung, einen Erntebehälter (3) zur Bioherstellung und eine Klärungseinrichtung (4) mit einer Zentrifuge (12) umfasst,
wobei der Quellbehälter (2) in einem zyklischen Herstellungsmodus betrieben wird,
wobei der zyklische Herstellungsmodus in dieser Reihenfolge die folgenden Schritte umfasst:
a) Beginnen des zyklischen Herstellungsmodus in dem Quellbehälter (2) mit anfänglichen Zellen (15) und Kultivierungsmedium (8),
b) Kultivieren der Zellen in dem Quellbehälter (2), dadurch Erhalten einer Zellbrühe (7), die kultivierte Zellen umfasst, wodurch der Quellbehälter (2) und das Kultivierungsmedium (8) dazu ausgestaltet sind, Quellkultur-Umgebungsbedingungen für die Kultivierung der Zellen bereitzustellen,
c) Ablassen einer Ablassfraktion (16) der Zellbrühe (7) von dem Quellbehälter (2),
d) Kombinieren einer Neubeginnfraktion (17) der Zellbrühe (7) mit frischem Kultivierungsmedium (8) und Wiederholen von Schritt b),
e) mindestens einmaliges Wiederholen der Schritte c) und d) und/oder
f) Ablassen der vom Schritt d) erhaltenen Zellbrühe (7) aus dem Quellbehälter (2), Anhalten des zyklischen Herstellungsmodus, Erhalten einer Ablassfraktion (16),
wobei das Verfahren ferner die folgenden Schritte umfasst:
i) Überführen der Ablassfraktion (16) zur Klärungseinrichtung (4) und Zentrifugieren der Ablassfraktion (16) über eine Zentrifuge (12), dadurch Trennen der Ablassfraktion (16) in mindestens eine zentrifugierte Ablassfraktion (19) und Überstand (14) und vorzugsweise Bioprodukt,
ii) Überführen mindestens eines Teils der zentrifugierten Ablassfraktion (19) von Schritt i) und frischen Kultivierungsmediums (8) in den Erntebehälter (3) und Betreiben des Erntebehälters (3) in einem Herstellungsmodus, wodurch der Erntebehälter (3) und das Kultivierungsmedium (8) dazu ausgestaltet sind, Erntekultur-Umgebungsbedingungen bereitzustellen, Herstellen des Bioprodukts in dem Erntebehälter (3),
wobei die Schritte i) und ii) mindestens zwei Mal mit Ablassfraktionen (16) von einer Ausführung von Schritt c) und/oder Schritt f) ausgeführt werden.

2. Verfahren nach Anspruch 1, **dadurch gekennzeichnet, dass** für mindestens eine, vorzugsweise mindestens zwei, bevorzugter jede Ausführung der Schritte b) und ii) die Kultivierung von Zellen in Schritt b) und die Bioherstellung in Schritt ii) zumindest teilweise parallel ausgeführt werden, dass vorzugsweise die Ausführungen der Schritte b) und ii) während mindestens 50 %, vorzugsweise mindestens 75 %, bevorzugter mindestens 90 %, der Zeit, die zur Kultivierung in Schritt b) verwendet wird, und/oder der Zeit, die zur Bioherstellung in Schritt ii) verwendet wird, und/oder der Zeit laufen, die zur parallelen Ausführung von Schritt b) und/oder Schritt ii) verwendet wird.

3. Verfahren nach Anspruch 1 oder 2, **dadurch gekennzeichnet, dass** ein einzelner Quellbehälterschlitz (20) mit einem einzelnen Erntebehälterschlitz (21) gepaart wird, dass Schritt b) während einer, insbesondere vorbestimmten, Kultivierungszeit ausgeführt wird, dass Schritt ii) während einer, insbesondere vorbestimmten, Herstellungszeit ausgeführt wird, dass die Kultivierungszeit und die Herstellungszeit und daher der Quellbehälterschlitz (20) und der Erntebehälterschlitz (21) derart synchronisiert werden, dass während mindestens zwei aufeinanderfolgenden Ausführungen, vorzugsweise sämtlichen Ausführungen, der Schritte ii) derselbe Erntebehälterschlitz (21) verwendet wird.

4. Verfahren nach Anspruch **3, dadurch gekennzeichnet, dass** die Schritte a) bis f) derart mit wiederholten Seed-Trains synchronisiert werden, dass nach dem Schritt f) der Schritt a) am selben Quellbehälterschlitz (20) wiederholt wird, ohne die Synchronität zwischen dem Quellbehälterschlitz (20) und dem Erntebehälterschlitz (21) zu unterbrechen.

5. Verfahren nach einem der vorhergehenden Ansprüche, **dadurch gekennzeichnet, dass** das Verfahren ferner den folgenden Schritt umfasst:
iii) Überführen einer Erntefraktion (27) von dem Erntebehälter (3) zur Klärungseinrichtung (4) und Zentrifugieren der Erntefraktion (27) über die Zentrifuge (12), wodurch das Bioprodukt von der Erntefraktion (27) getrennt wird,
dass vorzugsweise der Schritt iii) nach jedem Schritt ii) ausgeführt wird.

6. Verfahren nach einem der vorhergehenden Ansprüche, **dadurch gekennzeichnet, dass** die Bioprozessanlage (1) eine Sensoranordnung umfasst, dass die Sensoranordnung mindestens einen Sensor, der in oder an dem Quellbehälter (2) platziert ist, und/oder mindestens einen Sensor umfasst, der in oder an dem Erntebehälter (3) platziert ist, dass vorzugsweise mindestens ein Sensor dazu ausgestaltet ist, mindestens einen Prozessparameter aus der Gruppe von Konzentration der Kohlenstoffquelle, Konzentration der Stickstoffquelle, Aminosäurekonzentration, Wachstumsfaktorkonzentration, Sauerstoffkonzentration, Kohlendioxidkonzentration, pH-Wert, Temperatur, Leitfähigkeit, Druck, Biomassekonzentration, Biomasseherstellungsrate, Produktkonzentration, Produktivität, Sauerstoffaufnahmerate und/oder Rührdrehzahl für den Quellbehälter (2) und/oder für den Erntebehälter (3) zu messen.

7. Verfahren nach einem der vorhergehenden Ansprüche, **dadurch gekennzeichnet, dass** die Bioprozessanlage (1), insbesondere der Quellbehälterschlitz (20), eine elektronische Quellprozesssteuerung (22) zum Steuern der Umgebungsbedingungen der Quellkultur umfasst, dass vorzugsweise die elektronische Quellprozesssteuerung (22) mit dem mindestens einen Sensor verbunden ist, der in oder an dem Quellbehälter (2) platziert ist.

8. Verfahren nach einem der vorhergehenden Ansprüche, **dadurch gekennzeichnet, dass** Schritt ii) beendet wird, nachdem eine vorbestimmte Beendigungsbedingung erreicht wurde, dass vorzugsweise mindestens eine Sensormessung, welche die Beendigungsbedingung betrifft, wiederholt, insbesondere zyklisch oder ununterbrochen, durch den Sensor, der in oder an dem Erntebehälter (3) platziert ist, während mindestens einer, insbesondere jeder, Ausführung von Schritt ii) gemessen wird, dass bevorzugter die Sensormessung insbesondere durch eine elektronische Ernteprozesssteuerung (25) verwendet wird, um eine Zeit des Erreichens der Beendigungsbedingung vorherzusagen, dass die Quellkultur-Umgebungsbedingungen in Schritt b), insbesondere durch die elektronische Quellprozesssteuerung (22), gesteuert werden, um Schritt b) mit Schritt ii) zu synchronisieren.

9. Verfahren nach Anspruch 3 und 8, **dadurch gekennzeichnet, dass**, wenn eine Abweichung zwischen der vorhergesagten Zeit des Erreichens der Beendigungsbedingung und einem geplanten Ende der Herstellungszeit erkannt wird, insbesondere durch die elektronische Quellprozesssteuerung (22) oder die elektronische Ernteprozesssteuerung (25), eine Synchronisierungsstrategie ausgeführt wird, dass vorzugsweise die Synchronisierungsstrategie das Anpassen der Quellkultur-Umgebungsbedingungen und/oder Erntekultur-Umgebungsbedingungen zum Vermindern der resultierenden Abweichung zwischen dem Quellbehälter (2) und dem Erntebehälter (3), insbesondere zum Erreichen einer vordefinierten absoluten Zellmenge in dem Quellbehälter (2) vor dem Einleiten von Schritt c), umfasst.

10. Verfahren nach einem der vorhergehenden Ansprüche, **dadurch gekennzeichnet, dass** eine anfängliche lebensfähige Zellkonzentration in Schritt ii) höher als eine endgültige lebensfähige Zellkonzentration in Schritt b) ist, dass vorzugsweise die Zellkonzentration durch Zentrifugation in Schritt i) erhöht wird.

11. Verfahren nach einem der vorhergehenden Ansprüche, **dadurch gekennzeichnet, dass** mindestens ein Prozessparameter der Quellkultur-Umgebungsbedingungen, insbesondere anfänglichen Quellkultur-Umgebungsbedingungen, in mindestens einer, insbesondere jeder, Ausführung von Schritt b) und der Erntekultur-Umgebungsbedingungen, insbesondere anfänglichen Erntekultur-Umgebungsbedingungen, in mindestens einer, insbesondere jeder, Ausführung von Schritt ii) unterschiedlich eingestellt wird, dass vorzugsweise der mindestens eine Prozessparameter in den Quellkultur-Umgebungsbedingungen zur Kultivierung von Zellen über die Herstellung von Bioprodukt optimiert wird und/oder dass der mindestens eine Prozessparameter in den Erntekultur-Umgebungsbedingungen zur Herstellung von Bioprodukt über die Kultivierung von Zellen optimiert wird.

12. Verfahren nach einem der vorhergehenden Ansprüche, **dadurch gekennzeichnet, dass** die anfängliche Quellkulturumgebung, insbesondere die Quellkulturumgebung, und/oder die anfängliche Erntekulturumgebung, insbesondere die Erntekulturumgebung, für mindestens eine, insbesondere jede, Wiederholung von Schritt b) und/oder Schritt ii), im Wesentlichen jeweils identisch ist.

13. Verfahren nach einem der vorhergehenden Ansprüche, **dadurch gekennzeichnet, dass** das Kultivieren der Zellen in Schritt b) des zyklischen Herstellungsmodus unter Batch-Kultivierungsbedingungen oder Fed-Batch-Kultivierungsbedingungen durchgeführt wird, und/oder dass der Herstellungsmodus von Schritt ii) unter Fed-Batch-Kultivierungsbedingungen durchgeführt wird.

14. Verfahren nach einem der vorhergehenden Ansprüche, **dadurch gekennzeichnet, dass** die Zentrifuge (12) eine Fließbettzentrifuge (12) ist, dass vorzugsweise die Zentrifuge (12) in einem Vorwärtsbetrieb zur Zellseparation und/oder Zellwäsche und einem Rückwärtsbetrieb zum Zellablass betrieben wird, dass bevorzugter die Zentrifuge (12) in einem Rückwärtsbetrieb zum Überführen der zentrifugierten Ablassfraktion (19) zu dem Erntebehälter (3) zwischen den Schritten i) und ii) betrieben wird.

15. Verfahren nach einem der vorhergehenden Ansprüche, **dadurch gekennzeichnet, dass** nach dem Schritt c) ein Transfektionsschritt auf der Ablassfraktion (16) durchgeführt wird oder dass das Bioprodukt von den Zellen in dem Quellbehälter (2) hergestellt wird.

## Revendications

1. Procédé de fonctionnement d'une installation de bioprocédé (1) pour la production d'un bioproduit, dans lequel l'installation de bioprocédé (1) comprend un réceptacle de source (2) pour la culture de cellules, un réceptacle de récolte (3) pour la bioproduction et un dispositif de clarification (4) avec une centrifugeuse (12),
dans lequel le réceptacle de source (2) fonctionne dans un mode de production cyclique,
dans lequel le mode de production cyclique comprend, dans cet ordre, les étapes de :
a) lancement du mode de production cyclique dans le réceptacle de source (2) avec des cellules initiales (15) et du milieu de culture (8),
b) culture des cellules dans le réceptacle de source (2), ce qui donne ainsi un bouillon cellulaire (7) comprenant des cellules cultivées, moyennant quoi le réceptacle de source (2) et le milieu de culture (8) sont configurés pour fournir des conditions environnementales de culture de source pour la culture des cellules,
c) évacuation d'une fraction de décharge (16) du bouillon cellulaire (7) hors du réceptacle de source (2),
d) combinaison d'une fraction de redémarrage (17) du bouillon cellulaire (7) avec un milieu de culture frais (8) et répétition de l'étape b),
e) répétition des étapes c) et d) au moins une fois, et/ou
f) évacuation du bouillon cellulaire (7) obtenu à l'étape d) hors du réceptacle de source (2), arrêt du mode de production cyclique, obtention d'une fraction de décharge (16),
lequel procédé comprend en outre les étapes de :
i) transfert de la fraction de décharge (16) vers le dispositif de clarification (4) et centrifugation de la fraction de décharge (16) via la centrifugeuse (12), ce qui ainsi sépare la fraction de décharge (16) en au moins une fraction de décharge centrifugée (19) et un surnageant (14) et de préférence un bioproduit,
ii) transfert d'au moins une partie de la fraction de décharge centrifugée (19) obtenue à l'étape i) et de milieu de culture frais (8) dans le réceptacle de récolte (3) et fonctionnement du réceptacle de récolte (3) dans un mode de production, moyennant quoi le réceptacle de récolte (3) et le milieu de culture (8) sont configurés pour fournir des conditions environnementales de culture de récolte, et production du bioproduit dans le réceptacle de récolte (3),
dans lequel les étapes i) et ii) sont exécutées au moins deux fois avec les fractions de décharges (16) provenant de l'exécution de l'étape c) et/ou de l'étape f).

2. Procédé selon la revendication 1, **caractérisé en ce que** pour au moins une, de préférence au moins deux, mieux encore chacune des exécutions des étapes b) et ii), la culture de cellules dans l'étape b) et la bioproduction dans l'étape ii) sont exécutées au moins partiellement en parallèle, de préférence **en ce que** les exécutions des étapes b) et ii) prennent au moins 50 %, de préférence au moins 75 %, mieux encore 90 % du temps utilisé pour la culture dans l'étape b) et/ou du temps utilisé pour la bioproduction dans l'étape ii) et/ou du temps utilisé pour l'exécution de l'étape b) et/ou de l'étape ii) en parallèle.

3. Procédé selon la revendication 1 ou 2, **caractérisé en ce qu'**une fente unique de réceptacle de source (20) est appariée à une fente unique de réceptacle de récolte (21), **en ce que** l'étape b) est exécutée pendant un temps de culture prédéterminé particulier, **en ce que** l'étape ii) est exécutée pendant un temps de production prédéterminé particulier, **en ce que** le temps de culture et le temps de production et par conséquent la fente de réceptacle de source (20) et la fente de réceptacle de récolte (21) sont synchronisés de façon que pour au moins deux exécutions consécutives, de préférence toutes les exécutions, des étapes ii), la même fente de réceptacle de récolte (21) soit utilisée.

4. Procédé selon la revendication **3, caractérisé en ce que** les étapes a) à f) sont synchronisées avec des ensemencements répétés de façon qu'après l'étape f), l'étape a) soit répétée au niveau de la même fente de réceptacle de source (20) sans que la synchronicité entre la fente de réceptacle de source (20) et la fente de réceptacle de récolte (21) soit rompue.

5. Procédé selon l'une des revendications précédentes, **caractérisé en ce que** le procédé comprend en outre l'étape de
iii) transfert d'une fraction de récolte (27) du réceptacle de récolte (3) au dispositif de clarification (4), et centrifugation de la fraction de récolte (27) via la centrifugeuse (12), ce qui ainsi sépare le bioproduit d'avec la fraction de récolte (27),
de préférence **en ce que** l'étape iii) est exécutée après chaque étape ii).

6. Procédé selon l'une des revendications précédentes, **caractérisé en ce que** l'installation de bioprocédé (1) comprend un agencement de capteurs, **en ce que** l'agencement de capteurs comprend au moins un capteur placé dans le ou au niveau du réceptacle de source (2) et/ou au moins un capteur placé dans le ou au niveau du réceptacle de récolte (3), de préférence **en ce qu'**au moins un capteur est configuré pour mesurer au moins un paramètre de procédé du groupe comprenant la concentration de source de carbone, la concentration de source d'azote, la concentration d'acides aminés, la concentration de facteur de croissance, la concentration d'oxygène, la concentration de dioxyde de carbone, le pH, la température, la conductivité, la pression, la concentration de biomasse, le taux de production de biomasse, la concentration de produit, la productivité, le taux de captage d'oxygène et/ou la vitesse d'agitation pour le réceptacle de source (2) et/ou pour le réceptacle de récolte (3).

7. Procédé selon l'une des revendications précédentes, **caractérisé en ce que** l'installation de bioprocédé (1), en particulier la fente de réceptacle de source (20), comprend une commande de procédé électronique de source (22) pour commander les conditions environnementales de culture de source, de préférence **en ce que** la commande de procédé électronique de source (22) est connectée à l'au moins un capteur placé dans le ou au niveau du réceptacle de source (2).

8. Procédé selon l'une des revendications précédentes, **caractérisé en ce que** l'étape ii) est achevée après qu'une condition d'achèvement prédéterminée a été atteinte, de préférence **en ce qu'**au moins l'une mesure de capteur associée à la condition d'achèvement est mesurée de façon de façon répétée, en particulier de façon cyclique ou continue, par le capteur placé dans le ou au niveau du réceptacle de récolte (3) pendant au moins une, en particulier chaque, exécution de l'étape ii), mieux encore **en ce que** la mesure de capteur est utilisée, en particulier par une commande de procédé électronique de récolte (25), pour prédire le temps d'obtention de la condition d'achèvement, **en ce que** les conditions environnementales de culture de source dans l'étape b) sont commandées, en particulier par la commande de procédé électronique de source (22), pour synchroniser l'étape b) avec l'étape ii).

9. Procédé selon les revendications 3 et 8, **caractérisé en ce que** si un écart entre le temps prédit d'obtention de la condition d'achèvement et une fin planifiée du temps de production est détecté, en particulier par la commande de procédé électronique de source (22) ou la commande de procédé électronique de récolte (25), une stratégie de synchronisation est exécutée, de préférence **en ce que** la stratégie de synchronisation comprend l'adaptation des conditions environnementales de culture de source et/ou des conditions environnementales de culture de récolte pour réduire l'écart résultant entre le réceptacle de source (2) et le réceptacle de récolte (3), en particulier pour atteindre une quantité de cellules absolue prédéfinie dans le réceptacle de source (2) avant l'initiation de l'étape c).

10. Procédé selon l'une des revendications précédentes, **caractérisé en ce que** la concentration de cellules viables initiale dans l'étape ii) est supérieure à la concentration de cellules viables finale dans l'étape b), de préférence **en ce que** la concentration de cellules est augmentée par la centrifugation dans l'étape i).

11. Procédé selon l'une des revendications précédentes, **caractérisé en ce qu'**au moins un paramètre de procédé des conditions environnementales de culture de source, en particulier des conditions environnementales de culture de source initiales, dans au moins une, en particulier chaque, exécution de l'étape b) et des conditions environnementales de culture de récolte, en particulier des conditions environnementales de culture de récolte initiales, dans au moins une, en particulier chaque, exécution de l'étape ii), est établi différemment, de préférence **en ce que** l'au moins un paramètre de procédé est optimisé dans les conditions environnementales de culture de source pour la culture de cellules plutôt que la production de bioproduit et/ou **en ce que** l'au moins un paramètre de procédé est optimisé dans les conditions environnementales de culture de récolte pour la production de bioproduit plutôt que la culture de cellules.

12. Procédé selon l'une des revendications précédentes, **caractérisé en ce que** l'environnement de culture de source initial, en particulier l'environnement de culture de source, et/ou l'environnement de culture de récolte initial, en particulier l'environnement de culture de récolte, sont pratiquement identiques pour au moins une, en particulier chaque, répétition de l'étape b) et/ou de l'étape ii) respectivement.

13. Procédé selon l'une des revendications précédentes, **caractérisé en ce que** la culture des cellules dans l'étape b) du mode de production cyclique est effectuée dans des conditions de culture par lots ou des conditions de culture à écoulement discontinu, et/ou **en ce que** le mode de production de l'étape ii) est effectué dans des conditions de culture à écoulement discontinu.

14. Procédé selon l'une des revendications précédentes, **caractérisé en ce que** la centrifugeuse (12) est une centrifugeuse à lit fluidisé (12), de préférence **en ce que** la centrifugeuse (12) fonctionne en fonctionnement antérograde pour la séparation des cellules et/ou le lavage des cellules et en fonctionnement rétrograde pour la décharge de cellules, mieux encore **en ce que** la centrifugeuse (12) fonctionne en fonctionnement rétrograde pour transférer la fraction de décharge centrifugée (19) vers le réceptacle de récolte (3) entre les étapes i) et ii).

15. Procédé selon l'une des revendications précédentes, **caractérisé en ce qu'**une étape de transfection est effectuée après l'étape c) sur la fraction de décharge (16), ou **en ce que** le bioproduit est produit par les cellules dans le réceptacle de source (2).
